**Europäisches Patentamt**

**European Patent Office**

**Office européen des brevets**

(11) Veröffentlichungsnummer: **0 411 732 A1**

# (12) EUROPÄISCHE PATENTANMELDUNG

(21) Anmeldenummer: **90250197.2**

(22) Anmeldetag: **03.08.90**

(51) Int. Cl.5: **A61B 19/04**

(30) Priorität: **03.08.89 DE 3925938**

(43) Veröffentlichungstag der Anmeldung:
**06.02.91 Patentblatt 91/06**

(84) Benannte Vertragsstaaten:
**AT BE CH ES FR GB IT LI LU NL SE**

(71) Anmelder: **Keck, Claudia**
**Alt Wittenau 37 G**
**D-1000 Berlin 26(DE)**

(72) Erfinder: **Keck, Claudia**
**Alt Wittenau 37 G**
**D-1000 Berlin 26(DE)**

(74) Vertreter: **Wablat, Wolfgang**
**Patentanwalt Dr.-Ing. Dr. jur. Dipl.-Chem. W.**
**Wablat Potsdamer Chaussee 47**
**D-1000 Berlin 38(DE)**

(54) **Operationshandschuh.**

(57) Bei einem Operationshandschuh (1) werden an der Innenseite vorzugsweise zwischen zwei Schichten (5,6) aus Gummi - bzw. Kunststoffolie eingeschlossen - ein oder mehrere Indikatoren (4) oder ein Gemisch derselben zum Nachweis von Körperflüssigkeiten aufgebracht.

FIG.1

## OPERATIONSHANDSCHUH

Die Erfindung geht aus von einem Operationshandschuh aus Kunststoffolie oder Gummi gemäß dem Oberbegriff des Anspruchs 1 oder 3, wie er aus der DE-OS 2158409 bekannt ist.

Bekannte Operationshandschuhe, wie sie von Ärzten und Zahnärzten, Krankenschwestern und sonstigem medizinischen Personal benutzt werden, sind aus extrem dünnem Gummi oder anderen Kunststoffen hergestellt und derart eingerichtet, daß sie möglichst eng und dicht die Hand des Benutzers - ähnlich einer zweiten Haut - umschließen, um ein Maximum an Tastempfindlichkeit und Bewegungsfreiheit der Finger zu gewährleisten, während gleichzeitig der Übergang von Vergiftungs- oder Infektionskeimen von der nackten Haut des Benutzers zum Patienten und in umge -kehrter Richtung verhindert werden soll. Insbesondere Hepatitis- und HIV-Infektionen sollten in beiden Richtungen verhindert werden.

Ein wesentlicher Nachteil der bekannten Operationshandschuhe besteht darin, daß sie sehr leicht während der Benutzung durchlöchert werden oder zerreißen, doch wurde noch keine brauchbare Lösung dieses Problems gefunden. Aus der DE-OS 21 58 409 ist zwar ein Operationshandschuh bekannt, der Fingerlinge mit verstärkter Wand aufweist. Hierbei wird jedoch die Tastempfindlichkeit erheblich gesenkt. Das gleiche ist der Fall, wenn zur Vorsicht zwei oder drei Handschuhe übereinander getragen werden.

Auch ist es aus dieser Druckschrift bekannt, die Perforation durch den Austritt einer farbigen Flüssigkeit anzuzeigen.

Weiter ist die Möglichkeit bekannt, Perforationen anhand von Widerstandsmessungen nachzuweisen (FR - PS 2. 208. 300, US 4321 925, DE - GM 1900684).

Entscheidender Nachteil dieses Verfahrens ist, daß die Perforation nicht unmittelbar angezeigt werden kann.

Aufgabe der vorliegenden Erfindung ist es daher, den bekannten Handschuh so auszugestalten, daß ein Zerreißen oder auch schon eine Mikroperforation sicher frühzeitig erkannt wird.

Diese Aufgabe wird mit dem Handschuh nach dem Anspruch 1 oder 3 gelöst.Vorteilhafte Weiterbildungen der Erfindung sind in den Unteransprüchen angegeben. Nach dem heutigen Stand der Technik wird ein derartiger oder vergleichbarer Handschuh, auch nicht für andere Anwendungen hergestellt oder benutzt. Gemäß einer Ausgestaltung dieses Handschuhs erfolgt der Nachweis durch einen schnellen Farbumschlag. Nur solch schneller Nachweis macht ein unmittelbares Beseitigen pathogener Blut bzw. Speichelbestandteile möglich. Weitere Kontamination wird dann durch Handschuhwechsel verhindert.

Für den Handschuh benutzbare Indikatoren sind die bekannten Indikatoren, wobei für den Nachweis von Blut Indikatoren auf Basis von Guaiacum-Harz wegen der Abwesenheit cancerogener Stoffe bevorzugt werden. Bekannte Indikatoren für Blut sind beschrieben u. a. in den folgen Schriften : EP-OS 0 227 602, DE - OS 35 06 365, DE - AS 28 53 300 und CH - PS 427 353 sowie in "Fundamentals of Clinical Chemistry", Norbert W. Tietz, herausgegeben von W.B.Saunders Company, Philadelphia, 1982, Seiten 435 - 441.

Für den Speichelnachweis werden die aufgrund von Amylaseaktivität bekannten Indikatoren benutzt, wie sie z. B. in der GB - PS 1 489 059 beschrieben sind. Weitere Amylaseindikatoren werden auch in "Funadamentals of Clinical Chemistry", Norbert W. Tietz, Seiten 625 - 633 beschrieben.

In einem ersten Ausführungsbeispiel der Erfindung werden die Indikatoren für die Blut - und/ oder Speichelbestandteile - evtl. auf einem Träger - zusammen mit den üblich verwendeten Gleit- und Wasserabsorbtionsmitteln auf die Handschuhinnenfläche aufgebracht. Dies ist bei Gleitmitteln z. B. aus der DE - AS 24 39 134 bekannt. Typische Bei -spiele dafür sind Kaolin, synthetischer Quarz, Glas, Zellulose, mikrokristalline Zellulose, Calciumkarbonat, Magnesiumkarbonat, Aluminiumsilikat, Polyäthylenpuder und querver -netzte Maisstärke.

Diese Bestandteile können sowohl durch Aufstäubung als auch durch Einbettung auf die Innenseite der Handschuhe aufgebracht werden. Ein weiteres Ausführungsbeispiel besteht darin, bei der Herstellung der Handschuhe die Indikatoren zwischen zwei Schichten Gummi bzw.Kunststoffolie einzuschließen.

Das bevorzugte Verfahren zur Herstellung eines OP-Handschuhs wird im folgenden beschrieben: Zunächst wird eine Handschuhform, die den allgemeinen Umriß einer menschlichen Hand aufweist, in ein Tauchbad gebracht, welches mit einer geeigneten Masse aus Naturkautschuklatex oder anderen geeigneten gelösten Mischungen zur Handschuhfertigung gefüllt ist. Das Aufbringen der Außenschicht des Handschuhs wird auf bekannte Weise durch ein- oder mehrmaliges Tauchen der Form in die Masse durchgeführt, um die Schicht auf die gewünschte Dicke aufzubauen.

Nachdem die Schicht in geeigneter Weise auf die Form aufgebracht wurde, wird die Form aus dem Tauchtank genommen. Noch vor dem vollständigen Ausharten der Masse wird das im Anschluß beschriebene Indikatorgemisch entweder in Puderform, Dispersion oder Aufschwemmung in geeigneter Schichtdicke aufgebracht. Nach Aushärtung der Indikatorbeschichtung erfolgt ein abermaliges Eintauchen in die Naturkautschuklatexmasse oder in andere geeignete Mischungen. Nach dem Aufbau der gewünschten Schichtdicke wird die Form aus dem Tank entfernt. Es ergeben sich somit drei Schichten, die die Gesamtdicke des Handschuhs ergeben. Der Handschuh sollte nun so eingerichtet sein, daß die der Haut ferne Außenschicht am stärksten ausgebildet wird, die hautnahe Innenschicht sollte nur so stark sein, daß das Indikatorgemisch vollständig bedeckt ist und keinen Kontakt mit der Haut zuläßt, um zu vermeiden, daß eine ungleichmäßige Indikatorverteilung erfolgt oder durch Schwitzen der Hand eine Reaktion erfolgt. Im Bereich des Handgelenkes sollten die beiden Gummischichten über einige Zentimeter vulkanisiert bzw.verschweißt werden.Nach dem Trocknungsvorgang wird der Hanschuh von der Form gestreift und vorzugsweise gleich -zeitig gewendet. Geeignete mechanische Ausrüstungen zur industriellen Handschuhfertigung wie Transportvorrichtungen, Trocknungseinheiten usw. können bei der Durchführung des Verfahrens verwendet werden, werden hier jedoch im einzelnen nicht beschrieben und sind außerdem dem Fachmann bekannt.

Als Indikatorgemisch wird eine stabilisierte Mischung bevorzugt, die ein organisches Hydroperoxyd, einen Indikator (welcher durch katalytische Sauerstoffübertragung vom Hydroperoxyd zu einem Farbumschlag führt), einen Puffer (der die Zusammensetzung bei pH 4-7 hält) und ein Mittel zum Einkapseln der Komponenten enthält. Geeignete organische Peroxyde sind Cumolhydroperoxyd oder Diisopropylbenzol-hydroperoxyd. Als Indikator kommen die oben erwähnten Indikatoren, vorzugsweise Guajacol oder Guaiacum-Harz in Betracht. Der Puffer kann aus Citronensäure oder Natriumcitrat bestehen. Als Mittel zum Einkapseln werden Polysaccharide und deren Derivate benutzt.

Es ist ebenfalls möglich eine Zusammensetzung herzustellen, die aus zwei essentiellen Komponenten besteht. Einerseits wird eine Indikatorkomponente,z.B. Guariacum-Harz, auf einen Träger, wie z.B. Sorbit oder Mannit aufgebracht; dies geschieht entweder durch Aufsprühen oder in einem Wirbelbett apparat. Andererseits wird ein Träger mit einer Mischung aus Peroxyd (z.B. Bariumperoxyd) und einer sauren Komponente (z.B. Citronensäure oder Citratpuffer) imprägniert. Der Träger kann ebenfalls Sorbit bzw. Mannit sein oder auch Talkum, das als hochgereinigtes Magnesiumsilikat verwendet wird.

Beide Komponente werden gemischt und können in trockener Form weiterverarbeitet werden. Es wird darauf geachtet, daß in der gemischten Zusammensetzung der Guaiacum-Harz-Anteil zwischen 0.05 und 10 Gewichtsprozenten gehalten wird, da dieser Anteil zur optimalen Indikatoraktivität durch Farbumschlag führt. Um eine zu hohe Guaiacum-Harz Konzentration zu vermeiden können der Mischung inerte Stoffe wie z.B. nicht imprägniertes Talkum oder auch unbehandeltes Mannit bzw. Sorbit hinzugefügt werden.

In der Zeichnung ist ein bevorzugtes Ausführungsbeispiel für einen Operationshandschuh gemäß der Erfindung dargestellt. Es zeigen:

Fig. 1 eine teilweise aufgebrochene Ansicht des Operationshandschuhs,

Fig. 2 einen Schnitt durch den Operationshandschuh der Fig. 1 gemäß der Linie II-II,

Fig. 3 schematisch die Schichtstärken des Mehrschichten-Handschuhs

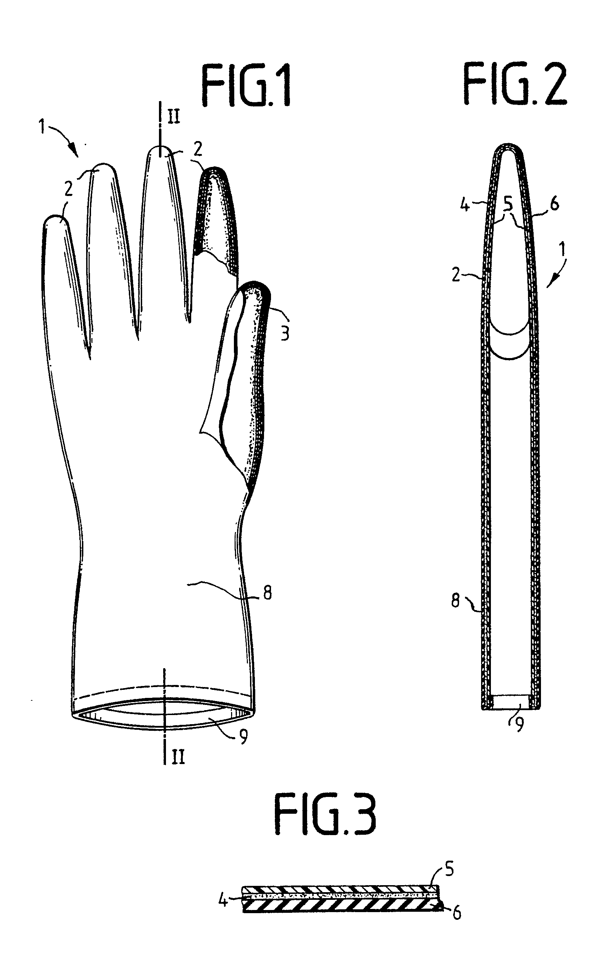

Die äußere Ansicht des dargestellten Finger-Handschuhs 1 gemäß Fig. 1 unterscheidet sich nicht von bekannten Operationshandschuhen.Der Handschuh hat vier Fingerköpfe 2 und einen Däumling 3. Der aufgebrochene Zeigefinger und der Daumen las sen an der Innenseite eine Schicht 4 erkennen, die als Indikator zum Nachweis von Korperflüssigkeiten dienen soll. Dabei können ein oder mehrere der oben beschriebenen Indikatoren oder ein Gemisch derselben zum Nachweis von Blut- oder Speichelbestandteilen dienen.

Der in Fig. 2 dargestellte Schnitt durch die Mittelfingerpartie gemäß der Linie II-II in Fig.1 zeigt jedoch den bedeutsamen Unterschied zum bekannten Operationshandschuh.Der neue Handschuh 1 besteht nämlich aus einer Innenschicht 5 und einer Außenschicht 6 aus Gummi oder Kunststoffolien. Zwischen den Handschuhschichten 5 und 6 befindet sich Indikatorschicht 4. Die Herstellung eines solchen Handschuhs erfolgt vorteilhaft nach dem ebenfalls oben beschriebenen Tauchverfahren, das erlaubt, die Stärke der einzelnen Schichten genau zu steuern.

Dabei ergeben sich, wie in Fig. 3 angedeutet, 3 Schichten, nämlich eine Innenschicht 5, eine Indikationsschicht 4 und eine Außenschicht 6. Die der Haut ferne Außenschicht 6 ist am stärksten ausgebildet, die hautnahme Innenschicht 5 dagegen nur so stark, daß das Indikatorgemisch 4 vollständig bedeckt ist und keinen Kontakt mit der Haut zuläßt.Die Gesamtstärke entspricht dabei etwa der Wandstärke eines normalen Operationshandschuhs,so daß der Tastsinn nicht beeinträchtigt wird. Die beiden Handschuhschichten 5 und 6 sind an der Handschuhstulpe 8 mit einem Bund 9 verschlossen.

Die oben beschriebenen Verfahrensweisen zur Herstellung des Handschuhs und die Beschreibung und Zusammensetzung der Indikatoren sind die bevorzugten. Vielfältige Ausführungen sind möglich und anwendbar. Alle anderen Ausführungen andern allerdings nichts an der vorliegenden Erfindung und werden von den nachfolgenden Ansprüchen miterfaßt.

Bezugszeichenliste

1. Handschuh
2. Fingerling
3. Däumling
4. Indikatorschicht
5. Innenschicht
6. Außenschicht
8. Handschuhstulpe
9. Bund

**Ansprüche**

1. Operationshandschuh bestehend aus Kunststoffolie oder Gummi, **dadurch gekennzeichnet,** daß an der Innenseite der Handschuhe ein oder mehrere Indikatoren oder ein Gemisch derselben zum Nachweis von Körperflüssigkeiten aufgebracht sind.

2. Operationshandschuh nach Anspruch 1, **dadurch gekennzeichnet,** daß die Indikatoren durch Aufstäubung oder Einbettung aufgebracht sind.

3. Operationshandschuh, bestehend aus zwei Schichten Gummi oder Kunstoffolie, **dadurch gekennzeichnet,** daß zwischen die Schichten (5,6) ein oder mehrere Indikatoren (4) oder ein Gemisch derselben zum Nachweis von Körperflüssigkeiten eingeschlossen sind.

4. Operationshandschuh nach Anspruch 3, **dadurch gekennzeichnet,** daß die der Haut ferne Außenschicht (6) am stärksten ausgebildet ist, während die hautnahe Innenschicht (5) nur so stark ist, daß das Indikatorgemisch (4) vollständig bedeckt ist.

5. Operationshandschuh nach einem der Ansprüche 1 bis 4, **dadurch gekennzeichnet,** daß als Körperflüssigkeiten Blut- oder Speichelbestandteile oder ein Gemisch von beiden nachgewiesen werden.

6. Operationshandschuh nach Anspruch 4, **dadurch gekennzeichnet,** daß durch die Körperflüssigkeiten ein Farbumschlag hervorgerufen wird.

7. Operationshandschuh nach Anspruch 1, **dadurch gekennzeichnet,** daß die Indikatoren zusammen mit Gleit- und / oder Wasserabsortionsmitteln aufgebracht sind.

# FIG.1

# FIG.2

# FIG.3

**Europäisches Patentamt**

**EUROPÄISCHER RECHERCHENBERICHT**

Nummer der Anmeldung

**EP 90 25 0197**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 905 449 (CUKIER)<br>* Figuren 2,3,4; Seite 8, Zeilen 8-11; Seite 10, Zeilen 33-34 *<br>– – – | 1-7 | A 61 B 19/04 |
| X | EP-A-0 320 541 (SEID)<br>* Spalte 5, Zeilen 28-35 *<br>– – – | 3-5 | |
| P,X | EP-A-0 368 456 (RICHARDSON)<br>* Spalte 9, Zeilen 54-57 *<br>– – – | 3-5 | |
| A | EP-A-0 300 814 (SURGIKOS)<br>* Ansprüche 1,8 *<br>– – – – – | 7 | |
| | | | **RECHERCHIERTE SACHGEBIETE (Int. Cl.5)**<br><br>A 61 B |

Der vorliegende Recherchenbericht wurde für alle Patentansprüche erstellt

| Recherchenort | Abschlußdatum der Recherche | Prüfer |
|---|---|---|
| Den Haag | 29 Oktober 90 | BARTON S.A. |